(19)

**Europäisches Patentamt**

**European Patent Office**

**Office européen des brevets**

(11) **EP 4 631 552 A1**

(12) **EUROPEAN PATENT APPLICATION**

(43) Date of publication:
**15.10.2025 Bulletin 2025/42**

(21) Application number: **24382365.5**

(22) Date of filing: **09.04.2024**

(51) International Patent Classification (IPC):
**A61M 11/00** *(2006.01)* **A61M 11/06** *(2006.01)*
**A61K 38/00** *(2006.01)* **A61M 35/00** *(2006.01)*
**A61L 27/00** *(2006.01)*

(52) Cooperative Patent Classification (CPC):
**A61M 15/0003; A61K 38/00; A61L 27/00;**
**A61M 11/007; A61M 11/06; A61M 35/00;**
A61M 15/0066

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
• **Universidad de Granada**
  **18071 Granada (ES)**
• **Universidad De Jaen**
  **23071 Jaen (ES)**
• **Universidade Da Coruña**
  **15071 A Coruña (ES)**

(72) Inventors:
• **MARCHAL CORRALES, Juan Antonio**
  **18016 Granada (ES)**
• **PLEGUEZUELOS BELTRÁN, Paula**
  **18016 Granada (ES)**
• **LÓPEZ RUIZ, Elena**
  **23071 Jaén (ES)**
• **NIETO GARCÍA, Daniel**
  **15071 A Coruña (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **SYSTEM FOR PROVIDING A BIOINK IN THE FORM OF A SPRAY**

(57)    The present invention relates to a system for providing a bioink in the form of a spray. The system comprises: a) at least two spray configured to receive at least two syringes comprising a nozzle each, each syringe configured to receive one or more components of the bioink; b) a housing, configured to comprise the at least two spray heads and comprising at least two housing nozzles, the housing nozzle comprising a diameter bigger than the diameter of the spray head nozzle; c) a gas inlet connected to the housing configured to receive a gas; and d) a control system comprising at least two valves, each valve associated to a housing nozzle and configured to control the flow of the gas through each housing nozzle. Each valve is further configured to control the provision of the one or more components of the bioink from each syringe, by controlling the flow of gas through its housing nozzle.

EP 4 631 552 A1

**Description**

## TECHNICAL FIELD

The present invention belongs to the field of medical devices. Particularly, the present invention relates to a system for providing a bioink in the form of a spray, and to a method of providing a bioink for the treatment of tissue injuries or damages using a system thereof.

## BACKGROUND OF THE INVENTION

Skin is one of the most important defense mechanisms in the body, protecting it against pathogens and chemical and physical hazards. The skin is composed of three main layers: the top layer is the epidermis, the middle and thicker layer is the dermis, and the subcutaneous tissue is the hypodermis. Skin wounds or injuries are breaks in the skin tissue, which may result from different causes, such as physical or chemical traumas (including burns or removal of skin during surgery), genetic irregularities, or skin diseases, such as epidermolysis bullosa, perianal fistulae or chronic wounds (like diabetic foot ulcers, pressure ulcers, and leg ulcers). Shallow injuries can usually be regenerated naturally by the skin's self-healing functions, but in deep wounds, the skin's regenerative components are destroyed, therefore requiring therapeutic interventions to facilitate wound healing.

Traditionally, the gold standard in clinical practice for treating deep wounds has been the use of autologous skin grafts (autografts). Despite their effectiveness, autografts present important limitations, such as the unavailability of sufficient donor skin in cases like major burn patients, and the creation of a secondary wound for the patient when harvesting said donor skin. Other grafting options include the culture of cells harvested from a small donor site to amplify their number, obtaining cultured epithelial autografts (CEAs), which require weeks to become available, or the temporary use of allografts or xenografts, which have the risk of diseases transmission and immune rejection.

On the other hand, thanks to the advances in regenerative medicine and tissue engineering, different biofabrication methods (such as 3D bioprinting or electrospinning) have been developed for the production of skin substitutes, which are defined as a heterogeneous group of substances (including hydrogels, cell suspensions, films, 3D scaffolds or cell sheets) intended to cover the wound and act as a barrier to avoid infections and fluid loss, promote wound healing, and reduce pain. Numerous cellular and acellular skin substitutes are commercially available for clinical use.

The biofabrication processes generally make use of bioinks, either acellular formulations containing biologically active components or molecules (but that can be subsequently seeded with cells afterward), or cell-based formulations that may also contain biomaterials and other biologically active components. To manufacture skin substitutes, bioinks have been formulated with various natural and synthetic polymeric biomaterials, that assemble into three-dimensional networks with a structure similar to the natural ECM, where cells can adhere, proliferate, differentiate, and promote skin regeneration. These biomaterials ought to have good mechanical properties, be biocompatible and biodegradable, be capable of maintaining humidity, and have enough porosity to allow cell migration and proliferation and the transport of nutrients and metabolic wastes. Some examples of biomaterials used for skin substitutes are collagen, fibrin, alginate, gelatin, glycosaminoglycans (GAGs), or chitosan.

One biofabrication technique disclosed in the prior art for application in skin regeneration is the use of skin sprays, which present several advantages for the delivery of bioinks or biomaterial inks to the wound bed: the possibility to treat large wounds or areas with unfavorable topography, in a rapid manner, with much more facility, and obtaining a homogeneous distribution of the sprayed material.

Acellular sprays generally consist of biomaterial inks that, when sprayed over the wound bed, create thin films or dressings covering the wounds, protecting them against TEWL and infections, and promoting the wound healing process. There are several acellular spray products that have been commercialized for decades now, the majority of them based on fibrin due to its hemostatic properties: for example, TISSEEL® and ARTISS® by Baxter [1,2], VISTASEAL™ by Johnson & Johnson [3], or Vivostat's Fibrin Sealant [4]. Other biomaterials that have been studied for acellular sprays are alginate [5], gelatin [6], chitosan [7], hyaluronic acid (HA) [8], and peptin [9].

On the other hand, cellular sprays apply different types of cell suspensions or bioinks. For *in vivo* studies, autologous epidermal cells (normally fibroblasts or keratinocytes) are mainly used, although allogeneic cells have also been studied. Once sprayed over the wound bed, epidermal cells can remain viable and proliferate, promoting re-epithelialization. Cell spray autografting consists in taking a biopsy of the patient's undamaged skin, digesting said biopsy, and obtaining a solution of autologous epidermal cells that can be sprayed immediately over the wound.

In contrast to traditional sheet autografts, cell sprays only require a much smaller donor area and offer a cost-effective, simple, and immediate treatment for the patient. Moreover, the harvested autologous cell can also be cultured to expand their number before being sprayed. There are many reports of cellular sprays being studied *in vitro* [10,11], in animals [12, 13] and in patients [14-17], for burns and wound healing applications, typically using airbrushes or syringes with spray nozzles as the spraying devices. However, few cell spray products have been approved for commercialization and use in clinical practice. The ReCell® kit by Avita Medical was the first "spray-on-skin" treatment approved by the U.S.

Food and Drug Administration (FDA) in 2018, and it employs an enzyme solution to isolate autologous epidermal cells from a small biopsy of the patient's healthy skin, which are then suspended in a lactate solution and sprayed over the wound using a syringe with a spray nozzle [18]. Similarly, RenovaCare developed another enzymatic isolation technique to obtain a solution of autologous epidermal cells from a biopsy of the patient, called the "CellMist Solution", which is delivered through their SkinGun, an electronically controlled pneumatic spray device. However, this product has not received approval for commercialization yet [19].

US2017304600A1 discloses a device and methods suitable for producing a cellular spray of cells for covering and growing cells on a damaged tissue, such as a skin wound. The cellular spray comprises tissue regenerating cells in a physiological solution with electrolytes which can be applied to a damaged tissue.

As mentioned above, despite their advantages, the translation of cell sprays to clinical practice is still limited and very few products have been approved for clinical use to date. There is therefore a need for providing novel spray device solutions that can be used in clinical practice that allows a simpler and easier to use way of providing bioinks at the will of the clinician according to the necessities of the tissue receiving the bioink spray.

## SUMMARY OF THE INVENTION

A first aspect of the invention relates to a system for providing a bioink in the form of a spray. The system comprises:

a) at least two spray heads, each a spray head configured to receive a syringe, each syringe configured to receive one or more components of the bioink and wherein each a spray head comprises a nozzle,
b) a housing (20), configured to comprise the at least two spray heads (12a, 12b), and comprising at least two housing nozzles (22a, 22b), each housing nozzle (22a, 22b) associated to a spray head (12a, 12b) and disposed around the spray head nozzle (125a, 125b), the housing nozzle (22a, 22b) comprising a diameter bigger than the diameter of the spray head nozzle (125a, 125b),
c) a gas inlet (30) connected to the housing (20) configured to receive a gas (300), and
d) a control system (40) comprising at least two valves (42a, 42b), each valve (42a, 42b) associated to a housing nozzle (22a, 22b) and configured to control the flow of the gas (300) through each housing nozzle (22a, 22b).

In this first aspect of the invention, each valve is further configured to control the provision of the one or more components of the bioink from each syringe through its spray head nozzle, by controlling the flow of gas through its housing nozzle, and the at least two valves are configured to provide the one or more components of the bioink of their respective syringe independently.

In a preferred embodiment of the first aspect of the invention, the at least two valves are configured to provide the one or more components of the bioink of their respective syringe sequentially and/or simultaneously.

In another preferred embodiment of the first aspect of the invention, each of the at least two valves is an electric valve and controlled through an actuator on the housing surface.

In another preferred embodiment of the first aspect of the invention, at least one of the at least two spray head nozzles comprises a diameter of 200µm and its associated housing nozzle comprises a diameter of 500 µm.

In another preferred embodiment of the first aspect of the invention, the at least two syringes are disposable syringes.

In another preferred embodiment of the first aspect of the invention, the housing comprises Acrylonitrile Butadiene Styrene (ABS).

In another preferred embodiment of the first aspect of the invention, the system further comprises a manometer to regulate the pressure the gas is provided through the gas inlet to the system.

In another preferred embodiment of the first aspect of the invention, the gas is provided with a pressure between 10 and 20 psi, preferably between 12 and 18 psi, more preferably 15 psi.

In another preferred embodiment of the first aspect of the invention, the system further comprises a filter between the gas inlet and the gas, preferably a membrane filter, more preferably a membrane filter of at most 0.22mm.

In another preferred embodiment of the first aspect of the invention, the viscosity of the bioink is comprised within 0.5 mPa·s and $10^4$ mPa·s.

In another preferred embodiment of the first aspect of the invention, the bioink is a bioink formulation comprising fibrinogen and a glycosaminoglycans (GAGs)/ collagen (Col) matrix, wherein the GAGs/Col matrix comprises at least

a) 60 - 75% (w/w) hyaluronic acid, and
b) 5 - 20% (w/w) sulphated GAGs, and
c) 1 - 5% (w/w) of collagen.

In a more preferred embodiment, the bioink formulation further comprises human cells, preferably human mesenchymal stem cells (hMSCs), keratinocytes and/or human dermal fibroblasts (hDFs).

In another more preferred embodiment, the amount of fibrinogen in the bioink formulation is:

(i) between 1 and 100 mg/ml, preferably about 10 mg/ml, and/or
(ii) between 0.1 and 10 % (w/v), preferably about 1% (w/v).

In another more preferred embodiment, the amount of GAGs/Col matrix in the bioink formulation is

(i) between 1 and 25 mg/ml, and/or
(ii) between 0.1 and 2.5% (w/v).

A second aspect of the invention relates to a method of providing a bioink for the treatment of tissue injuries or damages. The method comprises:

- providing at least two syringes (10a, 10b) each syringe (10a, 10b) comprising one or more components of the bioink (200) in system (100) according to any one of the embodiments of the first aspect of the invention, and
- actuating one or more of the at least two valves (42a, 42b) sequentially and/or simultaneously to provide the bioink (200) in the form of a spray over the tissue to be treated.

## BRIEF DESCRIPTION OF THE DRAWINGS

To enable a better understanding of the present disclosure, and to show how the present disclosure may be carried out, reference will now be made, by way of example only, to the accompanying schematic drawings, wherein:

Figure 1 shows a diagram of a system for providing a bioink in the form of a spray according to one or more embodiments of the invention.

Figure 2 shows a diagram of part of a system for providing a bioink in the form of a spray according to one or more embodiments of the invention.

Figure 3 shows an image of the at least two housing nozzles and at least two spray head nozzles and a detail of said nozzles according to one or more embodiments of the invention.

Figure 4 shows an image of a system for providing a bioink in the form of a spray according to one or more embodiments of the invention.

Figure 5 shows a diagram of a method of of providing a bioink for the treatment of tissue injuries or damages according to one or more embodiments of the invention.

Figure 6 shows the Metabolic activity, measured as fluorescence intensity (AU), of sprayed hDFs in FibD hydrogels at days 1, 2, 3, 5, and 7. Statistical significance: $*p < 0.05$.

Figure 7 shows a graphs for the physicochemical, rheological and mechanical characterization of a bioink according to one or more embodiments of the invention. (A) swelling and (B) degradation ratio of Fib and FibD hydrogels. (C) Viscosity of distilled water, 0.9% NaCl, and Fib and FibD inks. (D) Young's Modulus; and Viscoelastic moduli (E, Storage Modulus; F, Loss Modulus), of mice skin and hydrogels. Statistical significance: $*p < 0.05$; $**p < 0.01$; $***p < 0.005$.

## DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of', though less preferred.

When used herein "consisting of" excludes any element, step, or ingredient not specified in the claim element. When used herein, "consisting essentially of does not exclude materials or steps that do not materially affect the basic and novel characteristics of the claim.

As used herein, the term "glycosaminoglycan" or "GAG" refers to a long, unbranched, polysaccharide molecules found on the cell surface or extracellular matrix. Non-limiting examples of glycosaminoglycan include heparin, chondroitin sulfate, dextran sulfate, dermatan sulfate, heparan sulfate, keratan sulfate, crosslinked or non-crosslinked hyaluronic acid, hexuronyl hexosaminoglycan sulfate, and inositol hexasulfate. Derivatives, salts and mimetics of the above, including low molecular weight heparin are intended to be included in the invention.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application. Preferably, the term "treatment" refers to the application or administration of a pharmaceutical composition to a subject who has a disease or condition characterized by ER stress, with the purpose to cure, heal, alleviate, relieve, alter, remedy, ameliorate, improve, or affect the disorder, the symptom of the disease, or the predisposition toward a disease.

The terms "individual", "patient" or "subject" are used interchangeably in the present application and are not meant to be limiting in any way. The "individual", "patient" or "subject" can be of any age, sex and physical condition.

## DETAILED DESCRIPTION

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

A first aspect of the invention relates to a system for providing a bioink in the form of a spray.

The term "bioink" refers to a biomaterial formulated to mimic the characteristics of natural tissues. Based on its composition, bioinks can be classified into two primary types:

1) Cellular Bioinks or simply "bioinks", which refer to cell-based formulations that may also contain biomaterials and other biologically active components.
2) Acellular Bioinks or biomaterial inks: which refer to acellular formulations containing biologically active components or molecules (but that can be subsequently seeded with cells afterwards)..

The present invention is thus not limited to any particular type of bioink, and particularly, no discrimination is made between bioinks comprising cells and those not comprising cells, as both fall within the present definition of bioinks. Moreover, in the context of the present invention, the term bioink also comprises any bioink as above-defined in a hydrogel format.

The term "spray" refers to the dispersion of a substance in the form of fine liquid droplets, propelled under pressure or by other means into the air or onto a surface. The droplets are usually fine and are uniformly distributed over the target area, allowing for even coverage or distribution of the sprayed substance.

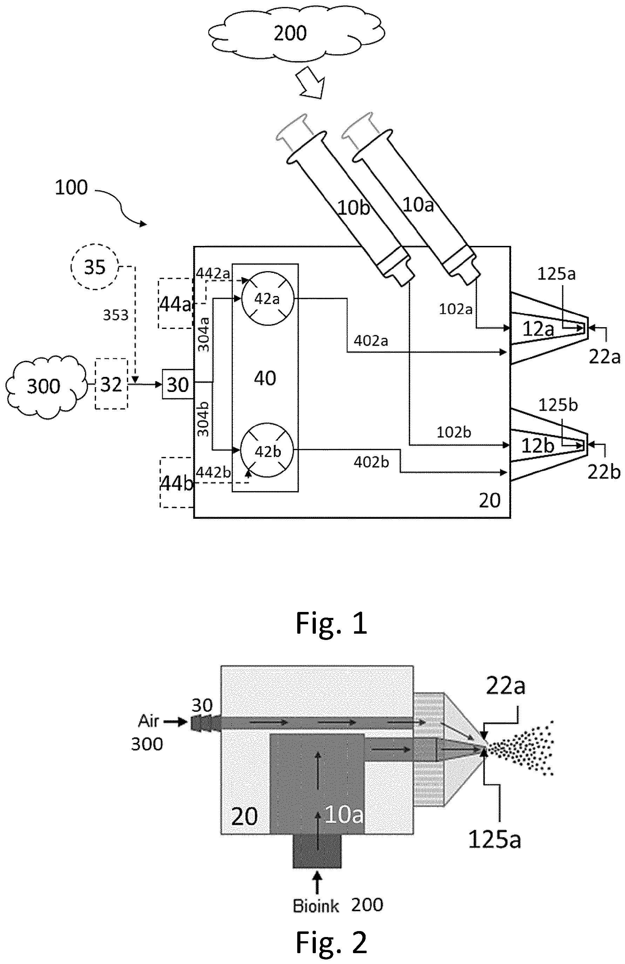

As shown with reference to Fig. 1 the system comprises at least two spray heads 12a, 12b each spray head (12a, 12b) configured to receive a syringe 10a, 10b, each syringe 10a, 10b configured to receive one or more components of the bioink 200 and wherein each a spray head (12a, 12b) comprises a nozzle 125a, 125b.

The term "syringe" refers to a device used to inject or withdraw fluids. It typically comprises a cylindrical barrel and a plunger. When the plunger is depressed, the fluid in the barrel is expelled through a tip or attached needle. The one or more syringes may be equal between them or may take different sizes, shapes, materials, for example, adapted to one or more components. For example, a syringe 10a may be bigger to accommodate a bigger volume when the one or more components its configured to receive is required in bigger quantities for the bioink the system 100 is configured to provide.

The term "spray head" refers to a device designed to disperse liquids, often in the form of fine droplets. Its primary function is to control the direction, volume, and characteristics of the spray.

The term "nozzle" refers to an opening designed to control the direction or characteristics of a fluid flow as it exits an enclosed chamber or pipe.

Each syringe 10a, 10b is configured to receive one or more components of the bioink (200), such that it is configured to accommodate or integrate the one or more components, but it does not necessarily mean that the one or more components are included within the syringe. The one or more components of the bioink (200), may in turn be also a bioink. Thus, each syringe 10a, 10b may be configured to receive a bioink (200'a, 200'b), wherein the different bioinks (200'a, 200'b), may in turn be components of the final bioink (200) to be provided.

While spray heads can vary significantly in design and function, a defining feature is the presence of a nozzle 125a, 125b. This nozzle 125a, 125b is what allows the spray head to effectively distribute the one or more components of the bioink in the form of a spray.

The spray head 12a, 12b may be configured to receive a syringe 10a, 10b different ways. This is represented through by arrows 102a and 102b. For example, the syringe 10a, 10b, may be directly attached, such that the syringe's tip or outlet might directly fit into the spray head, ensuring a seamless flow of fluid. Alternatively, the syringe and spray head may connect using a specially designed adapter or coupling that bridges the two, ensuring compatibility, such as quick-connect mechanisms or magnets. Also, a flexible hose or tube might connect the syringe to the spray head, allowing for distance or manoeuvrability between the two components. Another alternative involves.

The system 100 further comprises a housing 20, configured to comprise the at least two spray heads 12a, 12b, and comprising at least two housing nozzles 22a, 22b, each housing nozzle 22a, 22b associated to a spray head 12a, 12b and disposed around the spray head nozzle 125a, 125b, the housing nozzle 22a, 22b comprising a diameter bigger than the diameter of the spray head nozzle 125a, 125b.

The nozzle diameter is measured as the size of the aperture on the surface configured to exert the bioink. When the nozzle is circular, the diameter of the nozzle is the diameter of the circumference of the nozzle. When the nozzle is not circular, and takes other opening geometry, the diameter of the nozzle is the diameter of a circular nozzle with the similar hydrodynamic properties.

Each housing nozzle 22a, 22b is disposed around the spray head nozzle 125a, 125b such that the housing nozzle 22a, 22b and the spray head nozzle 125a, 125b define two nozzles, one within the other. In a preferred embodiment, the housing nozzle 22a, 22b and the spray head nozzle 125a, 125b are located in the same fluid provision axis.

The system 100, also comprises a gas inlet 30 connected to the housing 20 and configured to receive a gas 300. The gas inlet 30 serves as a crucial component for facilitating the entry of gas 300 into the system 100. This gas inlet 30 can be embodied through a variety of gas connection systems designed to ensure secure and efficient transfer of the gas 300, such as, threaded connectors, quick-connect/disconnect couplings, compression fittings, flange connectors, barbed connectors, bayonet connectors and magnetic couplings. The person skilled in the art may envisage many ways in which the gas inlet may be embodied to receive a gas. In some embodiments, the gas inlet 30 may comprise more than one physical inlet, for example, an inlet per housing nozzle, such that each gas inlet is configured to receive the gas associated to each housing nozzle.

The term "gas" within the context of the present disclosure is broad and encompasses any type of gaseous substance. This allows for flexibility in applications and ensures compatibility with a wide range of operations and requirements. In a preferred embodiment of the present disclosure, the gas 300 specified is air.

The system 100, further comprises a control system 40 comprising at least two valves 42a, 42b, each valve 42a, 42b associated to a housing nozzle 22a, 22b. Each valve 42a, 42b is configured to control the flow of the gas 300 through each housing nozzle 22a, 22b.

The control system 40, although represented as a rectangle in Fig. 1, is a sophisticated assembly designed to manage the system operation. It can integrate a myriad of components, spanning across the electronic, electric, and pneumatic domains. The skilled person may envisage many ways in which electronic, electric, and/or pneumatic elements may conform the control system 40 comprising at least two valves 42a, 42b, such that each valve 42a, 42b associated to a housing nozzle 22a, 22b is configured to control the flow of the gas 300 through each housing nozzle 22a, 22b.

The term "valve" refers to any mechanical or electromechanical device that regulates, directs, or controls the flow of a fluid (gases, liquids, fluidized solids, or slurries) by opening, closing, or partially obstructing one or more passageways.

The gas 300 may be connected from the gas inlet 30 to each valve 42a, 42b through one or more conduits 304a, 304b such as tubes.

Each valve 42a, 42b is associated to a housing nozzle 22a, 22b in the sense that each valve is configured to control the flow of the gas 300 through its associated housing nozzle 22a, 22b.

Each valve 42a, 42b is further configured to control the provision of the one or more components of the bioink 200 from each syringe 10a, 10b through its spray head nozzle 125a, 125b, by controlling the flow of gas 300 through its housing nozzle 22a, 22b. Each valve 42a, 42b may be connected to its housing nozzle 22a, 22b through one or more conduits 402a, 402b such as tubes.

A shown with respect to Fig. 2 since each housing nozzle 22a, 22b is associated to a spray head 12a, 12b, and each spray head 12a, 12b comprises a spray head nozzle 125a, 125b, each valve 42a, 42b is associated to a spray head nozzle 125a, 125b. Through the flowing of gas 300 through its housing nozzle 22a, 22b, the provision of the one or more components of the bioink 200 from each syringe 10a, 10b is controlled through a venturi effect on the spray head nozzle 125a, 125b which generates the spray of the one or more components of the bioink 200 as droplets propelled by the gas 300.

It is noted that for simplicity purposes, Fig. 2 shows only part of the system 100. Thus, Fig. 2 does not disclose a control system 40 nor at least two nozzle heads 12a, 12b, nor at least two housing nozzles 22a, 22b, although the system 100 comprises such elements.

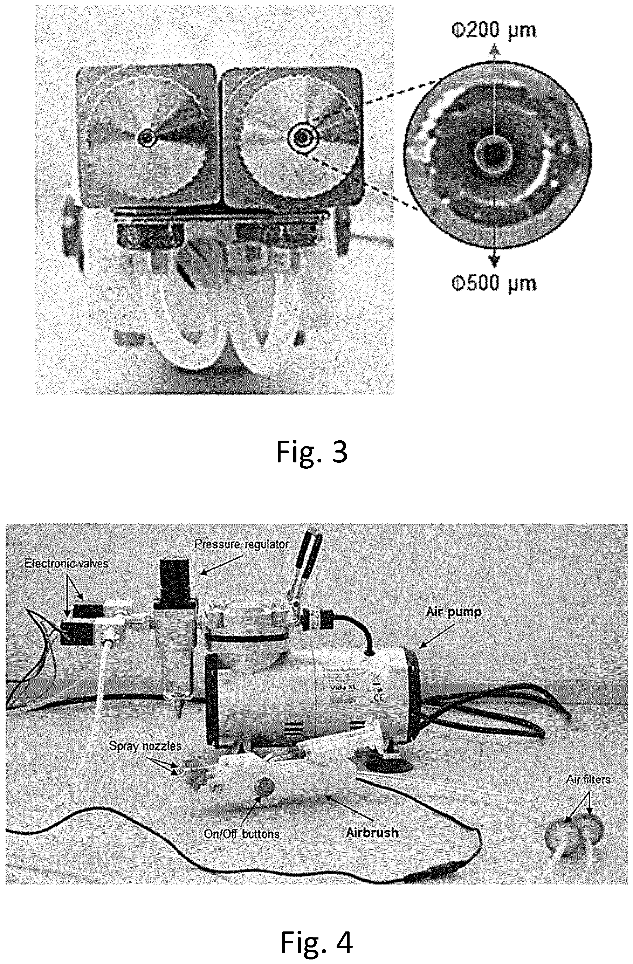

In a particular preferred embodiment, as shown with respect to Fig. 4, the system 100, further comprises the gas 300 and/or a gas source. The gas source may be a gas container, or an air pump. The person skilled in the art may envisage many different ways in which a gas source may be envisaged.

In a preferred embodiment of the first aspect of the invention, as shown with respect to Fig. 1, the at least two valves 42a, 42b are configured to provide the one or more components of the bioink 200 of their respective syringe 10a, 10b independently.

Since each valve 42a, 42b controls the provision of the one or more components of the bioink 200 from the syringe 10a, 10b configured to be connected to the spray head 12a, 12b, associated to the housing nozzle 22a, 22b, in turn associated to the valve 42a, 42b, the one or more components of the bioink 200 from any of the at least two syringes 10a, 10b can be provided independently.

Advantageously, this system provides a spray solution that enables its use in clinical practice, by enabling a simpler and easier to use way of providing different components of the bioink at the will of the clinician according to the necessities of the tissue receiving the bioink spray. For example, in some embodiments, a particular bioink may require the combination of a particular bioink with a substance that activates the bioink. Thus, the clinician may first provide the bioink in its inactive form as the first component of the bioink, and then provide the activation substance as the second component in a second stage, to activate the bioink once it has been provided.

In a more preferred embodiments, the at least two valves 42a, 42b are configured to provide the one or more components of the bioink 200 of their respective syringe 10a, 10b sequentially and/or simultaneously.

This further provides additional control on the provision of the one or more components of the bioink 200 which can further enable a simpler and easier to use way of providing the bioink 200 according to complex methodologies requiring sequential and/or simultaneous provision of one or more components of the bioink 200.

It is noted that Fig. 1 comprises many other elements, that are not comprised within the above-mentioned embodiments, and may be comprised in some preferred embodiments of the system 100, as will be described later on. For example, Fig. 1 shows references 32, 35, 44a, 44b. It is also noted that Figs. 1 and 2 are just schematical and the dimensions and shapes may differ in alternative embodiments of the above-mentioned embodiments of the first aspect of the invention. Moreover, Fig. 1 shows only two syringes 10a, 10b, but in other embodiments of the present invention the system may comprise more than two syringes. The same can be applied, mutatis, mutandis to the two spray heads 12a, 12b, and the two housing nozzles 22a, 22b.

In another preferred embodiment of the first aspect of the invention, and as shown with respect to Fig. 1, one or more of the at least two valves 42a, 42b is an electric valve and controlled through an actuator 44a, 44b on the housing 20 surface.

An electric valve may be understood as a valve that is controlled by an electric current. Its operation can be influenced by electronic management systems or might be based on electromechanical principles. The electric valve encompasses a wide range of valve types, from those purely driven by electric motors to those that integrate electronic circuits for more refined control, and even those utilizing electromechanical processes, where electrical power is transformed into mechanical actions to adjust the valve's position.

The actuator 44a, 44b may be any element actuatable by the user to control the one or more electric valves, such as buttons, pushbuttons, triggers, switches or the same. The skilled person may envisage many other alternatives for the actuator 44a, 44b at the light of this description, all of which are comprised within this disclosure. Each actuator may control one or more of the electric valves. For example, one actuator may control several valves simultaneously, while other actuator may control the operation of one single electric valve. Many different configurations may be foreseen at the light of this description, including actuators configured to actuate a plurality of valves in a predetermined manner. The actuator 44a, 44b may be electrically or mechanically connected to an electric valve 42a, 42b through a wire or mechanism 442a, 442b, such as a set of control wires, a trigger, a lever or any other mechanism the skilled person may envisage from the above disclosure.

Advantageously, the provision of one or more electric valves 42a, 42b controlled through one or more actuators 44a, 44b, simplifies the user interface and operational aspects of the system. Users can effortlessly decide which valve to actuate, ensuring a seamless and user-friendly experience. This approach provides enhanced precision and responsiveness in controlling the flow of gases through the valves, leading to more efficient and effective system operations.

In a more preferred embodiment, all of the at least two valves 42a, 44b, are electric valves.

In another preferred embodiment of the first aspect of the invention, and as shown for example in Fig. 3, at least one of the at least two spray head nozzles 125a, 125b comprises a diameter of 200µm and its associated housing nozzle 22a, 22b comprises a diameter of 500 µm.

Advantageously, as shown in Example 1, the provision of a spray head nozzle 125a, 125b with a diameter of 200µm and its associated housing nozzle 22a, 22b a diameter of 500 µm, provides a desired configuration for the provision of a bioink associated to tissue regeneration.

It is noted that according to other embodiments of this preferred embodiment, the at least one of the at least two spray head nozzles 125a, 125b comprises a diameter of 200µm ± 15%, and its associated housing nozzle 22a, 22b comprises a diameter of 500 µm ± 15%. More preferably, the at least one of the at least two spray head nozzles 125a, 125b comprises a diameter of 200µm ± 10%, and its associated housing nozzle 22a, 22b comprises a diameter of 500 µm ± 10%. Even further preferably, at least one of the at least two spray head nozzles 125a, 125b comprises a diameter of 200µm ± 5%, and its associated housing nozzle 22a, 22b comprises a diameter of 500 µm ± 5%.

Advantageously, the provision of a spray head nozzle 125a, 125b with a diameter of 200µm ± 15%, preferably ± 10%, more preferably ± 5%; and its associated housing nozzle 22a, 22b a diameter of 500 µm ± 15%, preferably ± 10%, more preferably ± 5%, provides a desired configuration for the provision of a bioink associated to tissue regeneration.

In another preferred embodiment of the first aspect of the invention, the at least two syringes 10a, 10b are disposable syringes.

The disposable syringes are single-use syringes designed to be used once and then discarded. They are typically made from plastic materials and come in various sizes and capacities to accommodate different applications.

Advantageously, this enables a bigger versatility, so that the system is readily adaptable when there is a need to change the components of the bioink. Also, since disposable syringes can come pre-filled, which eliminates the need for the user to handle bioink directly, reducing the potential for error and streamlining the process. Moreover, disposable syringes guarantee a sterile environment, minimizing the risk of contamination and ensuring compliance with stringent medical safety standards.

In another preferred embodiment of the first aspect of the invention, the housing 20 comprises Acrylonitrile Butadiene Styrene (ABS). Advantageously, the housing can be easily manufactured through 3D-printing techniques which in turn facilitates the creation of ergonomic and ad-hoc shape products that encompass the present invention.

In another preferred embodiment of the first aspect of the invention, and as shown with respect to Figs. 1 and 4, the system 100 further comprises a manometer 35 to regulate the pressure the gas 300 is provided through the gas inlet 30 to the system 100. The manometer may be connected to the gas 300 pipeline connected to the gas input 30 through a valve or regulator 353, or may be directly in series between the gas 300 and the gas input 32. While Fig. 1 shows the manometer 35 connected between a filter 32 and the gas input 30, the manometer 35 may be connected before the filter 32.

Advantageously, by adjusting and monitoring the pressure of the gas 300 entering through the gas inlet 30, it ensures that the bioink is delivered under optimal conditions, maintaining its integrity and efficacy of the bioink components. As shown in Example 1 it has been shown that controlling the gas 300 provision pressure is essential for some processes where bioinks are required.

In a more preferred embodiment of the first aspect of the invention, the gas 300 is provided with a pressure between 10 and 20 psi. As shown in Example 1, a gas 300 with a pressure between 10 and 20 psi is needed for an appropriate bioink spray formation. A pressure higher than 20psi, leads to less cell viability, while a pressure lower than 10psi is insufficient to maintain a constant and steady flow of the sprayed material.

In a further preferred embodiment, the gas 300 is provided with a pressure between 12 and 18 psi, more preferably at 15 psi.

In another preferred embodiment of the first aspect of the invention, the system 100 further comprises a filter 32 between the gas inlet 30 and the gas 300.

Advantageously this ensures the sterility of the gas provided. Gases such as air, can develop microorganism that, if mixed with the bionic, can transform the spray into an infection vector. Since the bioink may be used with therapeutic uses, such as substituting or treating an affected surface, it is desirable to ensure that the gas driving the bioink over the surface is sterile. The filter 32 effectively serves as an additional safety layer, which can further configure the system 100 for medical applications.

In a more preferred embodiment of the first aspect of the invention, the filter 32 is a membrane filter. Even more preferably, the filter is a membrane filter of at most 0.25mm, even more preferred at most 0.22mm. The size of the membrane indicates the smallest size of particles that the membrane filter is capable of filtering. Advantageously, a membrane filter of at most 0.22mm ensures the housing 20 receives a sterile gas 300 through the gas inlet 30.

In another preferred embodiment of the first aspect of the invention, the viscosity of the bioink (200) is comprised within 0.5 mPa·s and $10^4$ mPa·s.

Advantageously, a bioink 200 with a viscosity comprised between 0.5 mPa s and $10^4$ mPa·s is optimally sprayed using a device according to one or more embodiments of the first aspect of the invention.

In a more preferred embodiment, the bioink 200 comprises Fib and/or FibD, the viscosity of the bioink (200) is comprised between 1.163 $\pm$ 0.095 mPa·s for the Fib bioink; and/or from 1,114.75 mPa·s at shear rate y=0.1 $s^{-1}$, to 2.97 mPa·s at shear rate y=800 $s^{-1}$ for the FibD bioink; at 25°C.

The symbol "$\gamma$" is the shear rate of each material. Fib has a Newtonian fluid behaviour, so the viscosity is kept constant (1.163 $\pm$ 0.095 mPa s) at different shear rates. However, FibD has a shear-thinning fluid behaviour, so its viscosity decreases with higher shear-rates, thus the viscosity of FibD is presented as a range, depending on the shear rate.

In another preferred embodiment of the first aspect of the invention, the bioink 200 is a bioink formulation comprising fibrinogen and a glycosaminoglycans (GAGs)/collagen (Col) matrix, wherein the GAGs/Col matrix comprises at least

    a) 60 - 75% (w/w) hyaluronic acid, and
    b) 5 - 20% (w/w) sulphated GAGs, and
    c) 1 - 5% (w/w) of collagen.

Advantageously, a bioink with a formulation comprising fibrinogen, a GAGs/Col matrix and hMSCs and/or hDFs, wherein the GAGs matrix comprises at least 60 to 75% (w/w) hyaluronic acid, 5 to 20% (w/w) sulphated GAGs, and 1 to 5% (w/w) of collagen is the treatment of cutaneous wounds as described in Example 2-.

To this end two fibrinogen inks were formulated, one based on fibrinogen alone (Fib), and the other based on fibrinogen supplemented with GAGs/col matrix (FibD) as further detailed in Example 2. The ink is a source of GAGs/Col containing a high concentration of HA (67%), as well as sulfated GAGs (12%, including dermatan sulfate and chondroitin sulfate) and collagen (Col) (10.4%). These two inks were loaded with human mesenchymal stem cells (hMSCs) and human dermal fibroblasts (hDFs). The physicochemical properties of the bioinks, as well as their biocompatibility *in vitro*, were analyzed and found highly suitable for their intended application for spray application to the skin. Finally, their good skin wound healing properties were confirmed in an *in vivo* excisional wound healing murine model.

As detailed in Example 3 a), the Fib and FibD bioinks of present invention presented acidic pH values of 6.12 $\pm$ 0.19 and 6.10 $\pm$ 0.17, respectively, adequate for their topical application. The swelling or liquid absorption ability of hydrogels influences the entrance of fluids and the transport of nutrients into the hydrophilic hydrogel matrices. The Fib and FibD hydrogels presented a satisfactory swelling capacity after just two days (Example 3 b)). The FibD hydrogels showed a higher swelling ratio in comparison to the Fib hydrogels, expected due to the presence of high concentrations of HA in the GAGs/Col matrix component, given the high-water retention capability of HA.

On the other hand, the biodegradability of the hydrogels is important as it affects cell growth and tissue regeneration. As detailed in Example 3 c) the Fib hydrogels degraded faster than FibD hydrogels, probably owing to the more complex matrix formed in the FibD hydrogels as a result of the different biomolecules of GAGs/Col matrix. All Fib hydrogels had degraded completely after 17 weeks, whereas FibD hydrogels degraded after 20 weeks (i.e. Fib hydrogels degraded 15% faster than the FibD hydrogels).

In biofabrication, a high viscosity of the bioink or biomaterial ink is essential to maintain the 3D structure of the resulting construct, and specifically in skin sprays, to prevent run off of the sprayed material and prolong its contact time with the wound bed; but as mentioned previously, a higher viscosity of the transporting fluid affects cell viability negatively. Fibrinogen inks have been reported to exhibit a Newtonian fluid behavior, which is congruous with the results, where the Fib ink maintained a constant viscosity of 1.163 $\pm$ 0.095 mPa·s (Example 3 d)). On the other hand, the supplementation of the Fib ink with GAGs/Col matrix resulted in a shear-thinning behavior of the FibD ink. This shear-thinning behavior is favorable for the use of bioinks in biofabrication techniques such as skin sprays or 3D bioprinting, given that the decrease in viscosity under high shear stress (during extrusion) reduces cell damage, and the subsequent recovery of high viscosity under low shear stress (after extrusion) helps to prevent leakage of the material and maintain the 3D shape.

In a particular embodiment, the bioink 200 is a bioink formulation further comprises human cells, preferably human mesenchymal stem cells (hMSCs), keratinocytes and/or human dermal fibroblasts (hDFs).

In a more preferred embodiment of the first aspect of the invention, the amount of fibrinogen in the formulation is between 1 and 100 mg/ml. Additionally or alternatively, the amount of fibrinogen in the formulation is between 0.1 and 10 % (w/v). This amount of fibrinogen provides adequate viscosity and mechanical properties for the application, preferably as a spray, of the bioink onto the surface. Furthermore, the amount of fibrinogen allows, once mixed with thrombin, the formation of hydrogels in which the fibrin fibers form a matrix with a good density and porosity to allow the correct attachment, proliferation and viability of the cells.

In one embodiment the amount of fibrinogen in the formulation is between 2 and 90 mg/ml, between 3 and 80 mg/ml, between 4 and 70 mg/ml, between 5 and 60 mg/ml, between 6 and 50 mg/ml, between 7 and 40 mg/ml, between 8 and 30 mg/ml, between 9 and 20 mg/ml. In a preferred embodiment of the bioink formulation of present invention the amount of fibrinogen is about 10 mg/ml.

In one embodiment of the bioink formulation of present invention the amount of fibrinogen in the formulation is between 0.1 and 10% (w/v), between 0.2 and 9% (w/v), between 0.3 and 8% (w/v), between 0.4 and 7% (w/v), between 0.5 and 6% (w/v), between 0.6 and 5% (w/v), between 0.7 and 4% (w/v), between 0.8 and 3% (w/v), between 0.9 and 2% (w/v). In a preferred embodiment of the bioink formulation of present invention the amount of fibrinogen is about 1% (w/v).

In another more preferred embodiment of the first aspect of the invention, the bioink formulation of present invention the amount of GAGs/Col matrix in the formulation is between 1 and 25 mg/ml. Additionally or alternatively, the amount of GAGs/Col matrix in the formulation is between 0.1 and 2.5% (w/v).

This amount of GAGs/Col matrix provides for higher viability and number of cells. Furthermore, the GAGs/Col matrix provides better rheological properties to the bioink, as in terms of viscosity, the fibrinogen only bioink (Fib) presents a Newtonian behaviour, while the fibrinogen/ GAGs/Col matrix (FibD) bioink shows a shear-thinning behaviour.

In one embodiment of the bioink formulation of present invention the amount of GAGs/Col matrix in the formulation is between 1.1 and 20 mg/ml, between 1.2 and 15 mg/ml, between 1.3 and 10 mg/ml, between 1.4 and 5 mg/ml, between 1.5 and 4 mg/ml, between 1.6 and 3 mg/ml.

In a preferred embodiment the amount of GAGs/Col matrix in the formulation is 1.7 mg/ml. In an even more preferred embodiment, the amount of GAGs/Col matrix in the formulation is 2.5 mg/ml.

In another more preferred embodiment of the first aspect of the invention, the amount of hyaluronic acid in the GAGs/Col matrix is between 65 and 70% (w/w), between 66 and 69% (w/w), between 67 and 68% (w/w). In a preferred embodiment the amount of hyaluronic acid in the GAGs/Col matrix is about 67% (w/w).

In another more preferred embodiment of the first aspect of the invention, the amount of collagen in the GAGs/Col matrix is between 5 and 15% (w/w), preferably between 8 and 12% (w/w). In a preferred embodiment the amount of collagen in the GAGs/Col matrix is between 9 and 11% (w/w), most preferred about 10% (w/w).

In another more preferred embodiment of the first aspect of the invention, the sulphated GAGs in the GAGs/Col matrix are selected from dermatan sulphate and/or chondroitin sulphate.

In another more preferred embodiment of the first aspect of the invention, the amount of sulphated GAGs in the GAGs/Col matrix is between 10 and 15% (w/w), between 11 and 14% (w/w), preferably between 11 and 13% (w/w), most preferred about 12% (w/w).

A second aspect of the invention relates to a method of proving a bioink 200 for the treatment of tissue injuries or damages.

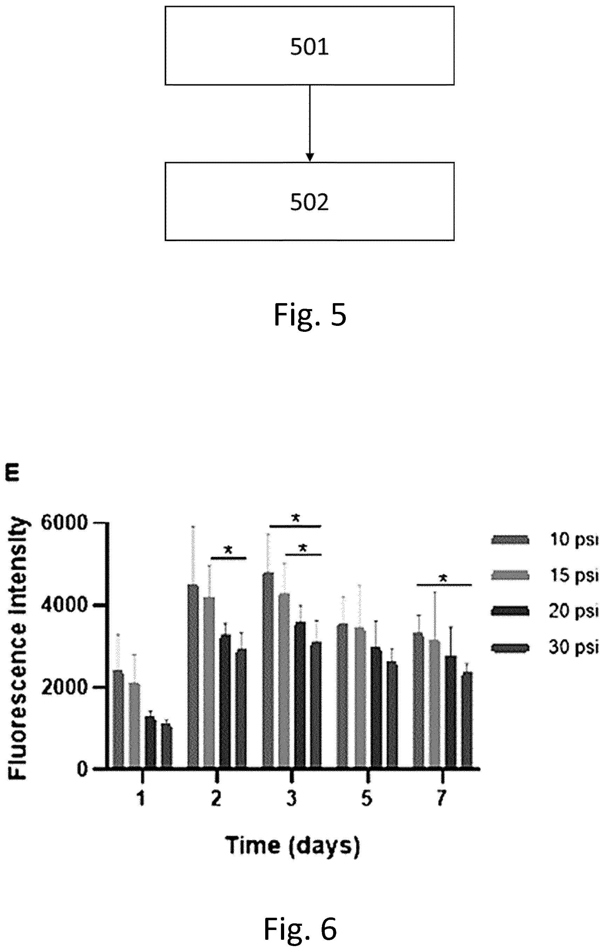

As shown in Fig. 5, the method comprises a first step 501 of providing at least two syringes 10a, 10b, each syringe 10a, 10b comprising one or more components of the bioink 200 in a system 100 according to any one of the embodiments of the first aspect of the invention.

A second step 502 involves actuating one or more of the at least two valves 42a, 42b sequentially and/or simultaneously to provide the bioink 200 in the form of a spray over the tissue to be treated.

Advantageously, the proposed method of providing a bioink 200 for the treatment of tissue injuries or damages allows for a method with higher control on the provision of the bioink, such that a particular bioink provision method can be followed, wherein each of the one or more components of the bioink can be provided sequentially and/or simultaneously. Therefore, one of the distinguishing advantages is the method's ability to independently supply individual bioink components. This flexibility ensures that the treatment can be precisely tailored to meet specific requirements.

For instance, consider a scenario in wound healing. The tissue might first require a layer from syringe 10a, which contains one or more bioink components that initiates cellular adhesion and establishes a primary scaffold. Once this layer is settled, a second set of one or more components from syringe 10b might then be sprayed to provide growth factors and stimulate cellular proliferation, accelerating the healing process. By controlling the sequence of component delivery, the method ensures optimal interaction of the components on the tissue, potentially optimizing therapeutic outcomes.

Also, there might be instances where a tissue needs immediate exposure to multiple bioink components to achieve the desired therapeutic outcome. In such cases, the method is equipped to deliver components from both syringes 10a and 10b simultaneously. An example might be a scenario where a tissue requires both structural support and immediate nutrient supply. The method's capacity to concurrently spray components ensures the tissue receives the combined benefits of both sets of one or more components of the bioink form the at least two syringes 10a, 10b without delay.

The method according to the second aspect of the invention holds promise for addressing various tissue injuries or damages. A prime example is skin damage. Whether it's due to burns, ulcers, abrasions, or surgical procedures, the skin often requires prompt and tailored treatment. Using the method described, clinicians can deliver specific bioink components, either sequentially or simultaneously, directly to the affected area. This ensures that the tissue receives exactly what it needs at the precise moment it's needed to effectively treat the tissue injury or damage. As previously discussed, the ability to control which bioink components are delivered, and in what order, allows for highly customized treatments. This is particularly crucial when treating complex wounds that might require a staged approach for optimal healing.

It is noted, that when the method is associated to a bioink formulation comprising fibrinogen, a glycosaminoglycans (GAGs)/Collagen (Col) matrix and optionally with human mesenchymal stem cells (hMSCs) and/or human dermal fibroblasts (hDFs), wherein the GAGs/Col matrix comprises at least 60 to 75% (w/w) hyaluronic acid, 5 to 20% (w/w) sulphated GAGs, and 1 to 5% (w/w) of collagen, the method is significantly suitable for treatment of the skin. As shown in Example 3, the physicochemical properties of the bioinks, as well as their biocompatibility *in vitro,* were analyzed and found highly suitable for their intended application for spray application to the skin. Finally, their good skin wound healing properties were confirmed in an *in vivo* excisional wound healing murine model.

All of the above are fully within the scope of the present disclosure, and are considered to form the basis for alternative embodiments in which one or more combinations of the above-described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## EXAMPLES

### Example 1: Spray device

A novel airbrush-based spraying device was designed to support dual extrusion, controlled by a pneumatic system (Figure 4). Placed on each side of the device, two on/off buttons (one for each material) connected to electronic valves open/close the airflow from the air pump (Figure 4). By pressing one of the On/Off buttons or both at the same time, both solutions can be sprayed either subsequently or simultaneously, respectively. A pressure regulator placed at the air pump controls air pressure, and consequently, the material deposition rate. By increasing the air pressure, more force is exerted, and a larger volume of air pushes the material out through the nozzle. The material is extruded through an inner 200 μm-diameter nozzle, while air is pushed through an outer 500 μm-diameter nozzle (Figure 3). When the material meets the air stream near the outlet orifice of the nozzles, the material is pulverized generating bioink droplets (Figure 2).

An AlamarBlue HS® assay was used to determine the best spraying pressure (Figure 6). Metabolic activity of hDFs decreased with increasing spraying pressure, evidencing how cells are damaged with increasing pressures. Although the lowest pressure (10 psi) showed the highest metabolic levels, this pressure proved to be insufficient to maintain a constant and steady flow of the sprayed material. Therefore, a spraying pressure of 15 psi was chosen for the following experiments as it still showed high metabolic activity levels, close to those of 10 psi.

Discussion

In this study, we have developed a novel spray system for the treatment of cutaneous wounds. To date, the vast majority of skin spray studies utilize syringes with spray pumps or regular airbrushes, which can only spray one material at a time; and the few spraying devices that allow the delivery of two materials (generally fibrinogen and thrombin solutions) are double syringes attached to spray tips. We have developed the first dual-extrusion airbrush-based device designed for the treatment of skin lesions, with a double spraying system that allows the delivery of two different materials either simultaneously or subsequently. Furthermore, the syringes can be quickly and easily replaced for others loaded with diverse bioinks, which could be useful in case of wanting to spray subsequent layers with different composition, in order to simulate the different layers of the skin. Given the numerous advantages for wound healing, our bioinks were formulated with fibrinogen, which has been the most widely used biomaterial for skin sprays due to its hemostatic properties, and supplemented with GAGs/Col matrix, which provides a source of GAGs, mainly HA but also dermatan sulfate, and collagen. In addition to the benefits of fibrinogen, HA promotes tissue regeneration during the wound healing process, reduces scar formation, maintains skin moisture, and has immunomodulatory properties. Moreover, GAGs/Col matrix has been reported to possess regenerative, anti-aging and antioxidant properties in vitro, and stimulate the synthesis of collagen, elastin, and GAGs. This is the first reported bioink based on a fibrinogen-HA combination for application in skin sprays.

A frequent drawback of many skin spray studies is the little importance they give to the spraying parameters, as the use of different parameters can influence results significantly, especially in cell sprays. Depending on the duration, cells are generally damaged by hydrostatic, shear, and elongation stresses while being sprayed and passing through the nozzle, but they are also damaged when they impact on the receiving surface. Cell viability has been reported to be negatively affected with a smaller nozzle diameter, higher spraying pressure and velocity, higher viscosity of the transporting fluid, and stiffness of the receiving surface. On the contrary, a larger cell-containing droplet diameter and a longer spraying distance are favorable for cell viability. Although the viability of epidermal cells after spraying has been demonstrated in several studies, cells can be damaged without having their membrane disrupted, so the post-aerosolization proliferative capacity must be assessed as well as the viability. Harkin et al. reported that the viability and mitochondrial enzyme activity of keratinocytes immediately after aerosolization was reduced with spraying pressures above 20 psi. We optimized the spraying pressure of our system to 15 psi, as higher pressures resulted in a decreased metabolic activity and proliferative capacity. For our experiments, we used an acetate cone as a funnel to keep better control of the sprayed volume. However, this would not be necessary in clinical use, as the area to be sprayed would generally be more extensive.

**Example 2: Formulation of fibrinogen-based bioinks**

Two different bioinks were formulated, one consisting of fibrinogen alone and another consisting of fibrinogen supplemented with GAGs/Col matrix. To prepare the fibrinogen (Fib) ink, bovine fibrinogen (Sigma-Aldrich) was diluted in 0.9% NaCl at 37°C, to obtain a final fibrinogen concentration of 10 mg/ml. Once dissolved, the solution was filtered through 0.22 $\mu$m membrane filters (Merck Millipore) to ensure sterility. For the fibrinogen- GAGs/Col matrix (FibD) ink, previously UV-sterilized GAGs/Col matrix was added to the Fib solution at a final concentration of 2.5 mg/mL. In case of preparing cell-loaded hydrogels, hMSCs or hDFs were then gently mixed with the Fib or FibD inks at a density of $1 \cdot 10^6$ cells/mL. As well, bovine thrombin (Sigma-Aldrich) was diluted in 40mM CaCh at a final concentration of 50 U/mL. Finally, the fibrin-based hydrogels were obtained by mixing the Fib or FibD bioinks with the thrombin solution in a 10:1 (v/v) ratio, respectively, and letting them gel for at least 5 minutes at 37°C.

**Example 3: Determination of Physicochemical properties**

a) pH determination

The pH of the Fib and FibD inks was measured (n=6) with a calibrated digital pH-meter Hach Sension+ (Hach Lange S.L., Spain) at room temperature. The pH values of the Fib and FibD inks were 6.12 $\pm$ 0.19 and 6.10 $\pm$ 0.17, respectively.

b) Swelling test of Fib and FibD hydrogels

Previously freeze-dried hydrogels were weighed, submerged in PBS, and kept at 37°C. At determined time points, samples (n=6) were taken out, absorbing excess of PBS with filter paper, and weighed. The swelling rates (%) were calculated as a measure of weight gain with the following equation:

$$Swelling\ ratio\ (\%) = \left(\frac{W_t - W_0}{W_0}\right) \times 100 \qquad (1)$$

$W_0$ is the initial wet weight of samples ($t = 0\ h$).
$W_t$ is the wet weight of samples at the corresponding time point (t).

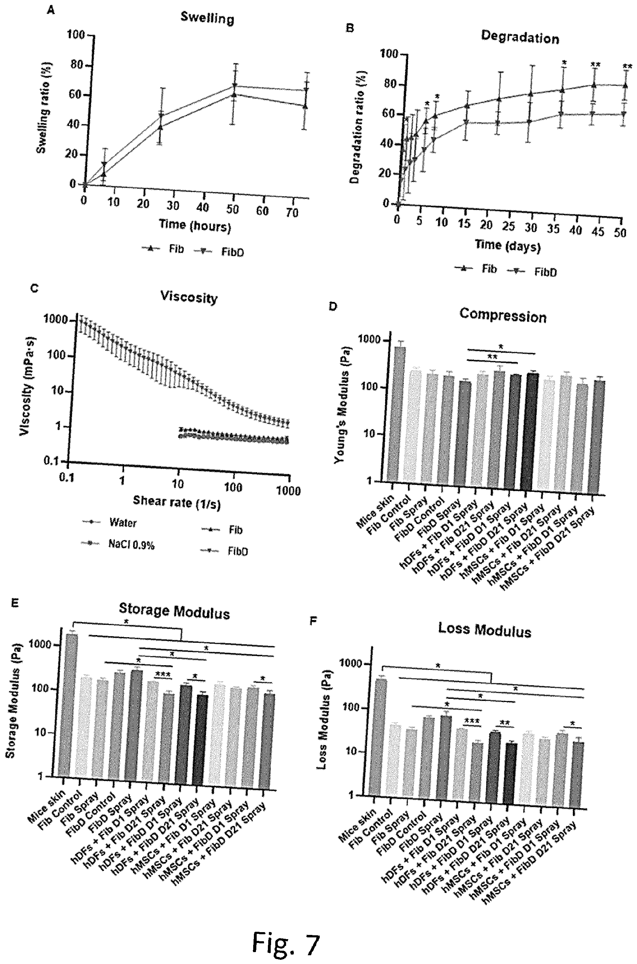

The swelling behavior of previously freeze-dried Fib and FibD hydrogels was observed by immersing the hydrogels in PBS and measuring weight increase (Figure 7A). For both Fib and FibD hydrogels, the swelling ratio increased during the first 48h, reaching an average swelling of 66.7 $\pm$ 10.2 % and 72.2 $\pm$ 5.0 % for Fib and FibD, respectively. Afterwards, hydrogels' weight started decreasing slightly due to degradation. The swelling ratio of the FibD hydrogels was higher than Fib at all time points, but no significant differences were observed.

c) Degradation test of Fib and FibD hydrogels

Pre-weighed hydrogels were immersed in PBS and maintained at 37°C under gentle agitation. At determined time intervals, samples (n=6) were centrifuged at 3000 G for 10 minutes, the supernatant was removed, and hydrogels were weighed. The degradation rates (%) as a measure of weight loss were calculated with the following equation:

$$Degradation\ ratio\ (\%) = \left(\frac{W_0 - W_t}{W_0}\right) \times 100 \qquad (2)$$

$W_0$ is the initial wet weight of samples ($t$ = 0 $h$).
$W_t$ is the wet weight of samples at the corresponding time point (t).

The degradation rate of hydrogels was analyzed by measuring weight loss over time (Figure 7B). The degradation of Fib and FibD hydrogels increased rapidly during the first week, and then kept increasing moderately every week. The Fib hydrogels degraded faster than the FibD hydrogels, presenting a higher degradation ratio during all the experiment. All Fib hydrogels had degraded completely after X days, whereas FibD hydrogels required X days to degrade.

d) Mechanical and rheological characterization

Rheological assays were carried out in order to obtain information on the viscosity of the fibrin-based inks, as well as on the compression and shearing characteristics of the hydrogels, using a torsional rheometer MCR302 (Anton Paar, Austria). All the rheological assays were performed in triplicate for all conditions and at a temperature of 25°C.

The shear viscosity was obtained using a cone-plate geometry (50 mm diameter and 1° angle). Samples of each material (distilled water, NaCl 0.9%, and the Fib and FibD inks) were sequentially placed on the base of the rheometer. First, they were rotatory pre-sheared at a constant shear rate of 800 s$^{-1}$ for 1 min. Then, they were allowed to rest for 1 min, with no shear rate applied. Lastly, they were rotatory sheared with a logarithmic shear rate ramp from 0.01 to 800 s$^{-1}$ (acquisition time of 5 seconds) for 5 min.

A plate-plate geometry (20 mm diameter and 5 mm gap) was used to analyze the compression and oscillatory shear behavior of hydrogels (prepared with a diameter of 20 mm and a height of approximately 5 mm) and mice skin samples (cut in a circular form with the same diameter, 20 mm). First, samples were placed onto the base of the rheometer and were approached by the rheometer head at a constant speed of 10 $\mu$m/s, up to a normal force of 0.1 N (in the case of the hydrogels) or 0.5 N (in the case of the mice skin), to determine the Young's modulus. Secondly, the normal force was kept constant for 30 seconds at 0.1 N or 0.5 N, correspondingly. Finally, to determine the shear viscoelastic moduli, the samples were oscillatory sheared according to a strain amplitude of 0.001% to 1000% at a frequency of 1 Hz and a normal force of 0.1 N (for hydrogels) or 0.5 N (for mice skin).

In order to assess the capability of the inks to be sprayed through the airbrush's nozzle, the shear viscosity of the Fib and FibD inks, as well as distilled water and 0.9% NaCl (the basis of the fibrin inks formulation), was determined with a rotational shear test (Figure 7C). At 25°C, water and NaCl obtained viscosity results of 0.871 $\pm$ 0.039 and 0.896 $\pm$ 0.272 mPa s, respectively. As expected, they demonstrated a Newtonian flow behavior, with viscosity being independent of the shear rate. Interestingly, despite being a polymeric solution, the Fib ink also showed a Newtonian behavior, with a viscosity of 1.163 $\pm$ 0.095 mPa s. However, the addition of GAGs/Col matrix (which contains high concentrations of HA molecules) resulted in the FibD ink having a shear-thinning behavior, with a decreasing viscosity with increasing shear rates (ranging from 1,114.75 mPa·s at $\gamma$˙=0.1 s-1, to 2.97 mPa·s at $\gamma$ ˙=800 s-1), typical of polymeric solutions.

On the other hand, the compression and oscillatory shearing characteristics of the hydrogels formed from the bioinks are analyzed in Figures 2D, E and F. Manually created (pipetted) Fib and FibD hydrogels (Control) were compared with sprayed Fib and FibD hydrogels, respectively, with no significant differences found. Between Fib and FibD hydrogels, differences were not statistically significant, but higher Storage (Figure 7E) and Loss (Figure 7F) moduli could be observed for the FibD hydrogels than the Fib ones, but this difference was not observable in cell-loaded hydrogels. There were significant differences in the Young's Modulus between sprayed acellular FibD hydrogels and sprayed hDFs-loaded FibD hydrogels, both after 1 and 21 days (Figure 7D); as well as in the Storage and Loss moduli between sprayed acellular Fib hydrogels and sprayed hDFs-loaded Fib hydrogels after 21 days in culture; and between sprayed acellular FibD hydrogels and hDFs-loaded FibD hydrogels after 21 days (Figures 2E and F). For hDFs and hMSCs-loaded hydrogels, both with Fib and FibD, differences were observed when comparing hydrogels maintained in culture for 1 or 21 days: hydrogels cultured for 21 days showed higher Young's moduli (Figure 7D) and lower Viscoelastic moduli (Figures 2E and F), than those cultured for 1 day. Finally, samples of healthy skin of mice revealed higher Young's and Viscoelastic moduli than all hydrogels, but the difference was not statistically significant for the Young's Modulus.

References

[1] TISSEEL, (n.d.). https://globaladvancedsurgery.baxter.com/tisseel (accessed October 18, 2022).

[2] ARTISS, (n.d.). https://globaladvancedsurgery.baxter.com/artiss (accessed October 18, 2022).

[3] VISTASEALTM Fibrin Glue & Sealant | Ethicon, (n.d.). https://www.jnjmedtech.com/en-US/product/vistaseal-fibrin-sealant-human (accessed October 18, 2022).

[4] Vivostat - Fibrin Sealant, (n.d.). https://vivostat.com/products/vivostat-fibrin-sealant (accessed October 18, 2022).

[5] O. Catanzano, M.C. Straccia, A. Miro, F. Ungaro, I. Romano, G. Mazzarella, G. Santagata, F. Quaglia, P. Laurienzo, M. Malinconico, Spray-by-spray in situ crosslinking alginate hydrogels delivering a tea tree oil microemulsion, European Journal of Pharmaceutical Sciences. 66 (2015) 20-28. https://doi.org/10.1016/j.ejps.2014.09.018.

[6] P.M. Neumann, B. Zur, Y. Ehrenreich, Gelatin-based sprayable foam as a skin substitute, J Biomed Mater Res. 15 (1981) 9-18. https://doi.org/10.1002/jbm.820150105.

[7] Y. Li, Q.Q. Leng, X.L. Pang, H. Shi, Y.L. Liu, S.S. Xiao, L. Zhao, P. Zhou, S.Z. Fu, Therapeutic effects of EGF-modified curcumin/chitosan nano-spray on wound healing, Regen Biomater. 8 (2021). https://doi.org/10.1093/RB/RBAB009.

[8] J. Chen, H. Wang, L. Mei, B. Wang, Y. Huang, G. Quan, C. Lu, T. Peng, X. Pan, C. Wu, A pirfenidone loaded spray dressing based on lyotropic liquid crystals for deep partial thickness burn treatment: healing promotion and scar prophylaxis, J Mater Chem B. 8 (2020) 2573-2588. https://doi.org/10.1039/C9TB02929J.

[9] K.M.G. Jáuregui, J.C.C. Cabrera, E.P.S. Ceniceros, J.L.M. Hernández, A. Ilyina, A new formulated stable papin-pectin aerosol spray for skin wound healing, Biotechnology and Bioprocess Engineering. 14 (2009) 450-456. https://doi.org/10.1007/s12257-008-0268-0.

[10] B. ter Horst, R.J.A. Moakes, G. Chouhan, R.L. Williams, N.S. Moiemen, L.M. Grover, A gellan-based fluid gel carrier to enhance topical spray delivery, Acta Biomater. 89 (2019) 166-179. https://doi.org/10.1016/j.actbio.2019.03.036.

[11] W.S. Veazey, K.U. Anusavice, K. Moore, Mammalian cell delivery via aerosol deposition, J Biomed Mater Res B Appl Biomater. 72 (2005) 334-338. https://doi.org/10.1002/jbm.b.30159.

[12] F.A. Navarro, M.L. Stoner, C.S. Park, J.C. Huertas, H.B. Lee, F.M. Wood, D.P. Orgill, Sprayed Keratinocyte Suspensions Accelerate Epidermal Coverage in a Porcine Microwound Model, Journal of Burn Care & Rehabilitation. 21 (2000) 513-518. https://doi.org/10.1097/00004630-200021060-00007.

[13] F.O.G. Fraulin, A. Bahoric, A.R. Harrop, T. Hiruki, H.M. Clarke, Autotransplantation of epithelial cells in the pig via an aerosol vehicle, Journal of Burn Care and Rehabilitation. 19 (1998) 337-345. https://doi.org/10.1097/00004630-199807000-00012.

[14] J.C. Gerlach, C. Johnen, C. Ottoman, K. Bräutigam, J. Plettig, C. Belfekroun, S. Münch, B. Hartmann, Method for autologous single skin cell isolation for regenerative cell spray transplantation with non-cultured cells, International Journal of Artificial Organs. 34 (2011) 271-279. https://doi.org/10.5301/IJAO.2011.6508.

[15] B. Hartmann, A. Ekkernkamp, C. Johnen, J.C. Gerlach, C. Belfekroun, M. v. Küntscher, Sprayed Cultured Epithelial Autografts for Deep Dermal Burns of the Face and Neck, Ann Plast Surg. 58 (2007) 70-73. https://doi.org/10.1097/01.sap.0000250647.39784.bb.

[16] P. Johnstone, J.S.S. Kwei, G. Filobbos, D. Lewis, S. Jeffery, Successful application of keratinocyte suspension using autologous fibrin spray, Burns. 43 (2017) e27-e30. https://doi.org/10.1016/j.burns.2016.05.010.

[17] R. Esteban-Vives, M.S. Choi, M.T. Young, P. Over, J. Ziembicki, A. Corcos, J.C. Gerlach, Second-degree burns with six etiologies treated with autologous noncultured cell-spray grafting, Burns. 42 (2016) e99-e106. https://doi.org/10.1016/j.burns.2016.02.020.

[18] AVITA MEDICAL, (n.d.). https://avitamedical.com/product/ (accessed October 13, 2022).

[19] Our Technology | RenovaCare, (n.d.). https://www.renovacareinc.com/technology/ (accessed October 17, 2022).

**Claims**

1. A system (100) for providing a bioink (200) in the form of a spray, the system comprising:

   a. at least two spray heads (12a, 12b), each a spray head (12a, 12b) configured to receive a syringe (10a, 10b), each syringe (10a, 10b) configured to receive one or more components of the bioink (200) and wherein each a spray head (12a, 12b) comprises a nozzle (125a, 125b),
   b. a housing (20), configured to comprise the at least two spray heads (12a, 12b), and comprising at least two housing nozzles (22a, 22b), each housing nozzle (22a, 22b) associated to a spray head (12a, 12b) and disposed around the spray head nozzle (125a, 125b), the housing nozzle (22a, 22b) comprising a diameter bigger than the diameter of the spray head nozzle (125a, 125b),
   c. a gas inlet (30) connected to the housing (20) configured to receive a gas (300), and
   d. a control system (40) comprising at least two valves (42a, 42b), each valve (42a, 42b) associated to a housing nozzle (22a, 22b) and configured to control the flow of the gas (300) through each housing nozzle (22a, 22b), wherein each valve (42a, 42b) is further configured to control the provision of the one or more components of the bioink (200) from each syringe (10a, 10b) through its spray head nozzle (125a, 125b), by controlling the flow of gas (300) through its housing nozzle (22a, 22b), and
   wherein the at least two valves (42a, 42b) are configured to provide the one or more components of the bioink (200) of their respective syringe (10a, 10b) independently.

2. The system (100) according to claim 1, wherein the at least two valves (42a, 42b) are configured to provide the one or more components of the bioink (200) of their respective syringe (10a, 10b) sequentially and/or simultaneously.

3. The system (100) according to any one of the previous claims, wherein each of the at least two valves (42a, 42b) is an electric valve and controlled through an actuator (44a, 44b) on the housing (20) surface.

4. The system (100) according to any one of the previous claims, wherein at least one of the at least two spray head nozzles (125a, 125b) comprises a diameter of 200µm and its associated housing nozzle (22a, 22b) comprises a diameter of 500 µm.

5. The system (100) according to any one of the previous claims, wherein the at least two syringes (10a, 10b) are disposable syringes.

6. The system (100) according to any one of the previous claims, wherein the housing (20) comprises Acrylonitrile Butadiene Styrene (ABS).

7. The system (100) according to any one of the previous claims, wherein the system (100) further comprises a manometer (35) to regulate the pressure the gas (300) is provided through the gas inlet (30) to the system (100).

8. The system (100) according to any one of the previous claims, wherein the gas (300) is provided with a pressure between 10 and 20 psi, preferably between 12 and 18 psi, more preferably 15 psi.

9. The system (100) according to any one of the previous claims, wherein the system (100) further comprises a filter (32) between the gas inlet (30) and the gas (300), preferably a membrane filter, more preferably a membrane filter of at most 0.22mm.

10. The system (100) according to any one of the previous claims, wherein the viscosity of the bioink (200) is comprised within 0.5 mPa·s and $10^4$ mPa s.

11. The system (100) according to any one of the previous claims, wherein the bioink (200) is a bioink formulation comprising fibrinogen and a glycosaminoglycans (GAGs)/ collagen (Col) matrix, wherein the GAGs/Col matrix comprises at least

    a) 60 - 75% (w/w) hyaluronic acid, and

b) 5 - 20% (w/w) sulphated GAGs, and

c) 1 - 5% (w/w) of collagen.

12. The system (100) according to claim 11, wherein the bioink formulation further comprises human cells, preferably human mesenchymal stem cells (hMSCs), keratinocytes and/or human dermal fibroblasts (hDFs).

13. The system (100) according to any one of claims 11 or 12, wherein the amount of fibrinogen in the bioink formulation is

(i) between 1 and 100 mg/ml, preferably about 10 mg/ml, and/or

(ii) between 0.1 and 10 % (w/v), preferably about 1% (w/v).

14. The system (100) according to any one of claims 11 to 13, wherein the amount of GAGs/Col matrix in the bioink formulation is

(i) between 1 and 25 mg/ml, and/or

(ii) between 0.1 and 2.5% (w/v).

15. Method of providing a bioink (200) for the treatment of tissue injuries or damages, wherein the method comprises:

- providing at least two syringes (10a, 10b) each syringe (10a, 10b) comprising one or more components of the bioink (200) in system (100) according to any one of claims 1 to 14, and
- actuating one or more of the at least two valves (42a, 42b) sequentially and/or simultaneously to provide the bioink (200) in the form of a spray over the tissue to be treated.

# Fig. 1

# Fig. 2

Fig. 3

Fig. 4

Fig. 5

Fig. 6

EP 4 631 552 A1

Fig. 7

Europäisches
Patentamt

European
Patent Office

Office européen
des brevets

## EUROPEAN SEARCH REPORT

**Application Number**

EP 24 38 2365

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 179 862 B1 (SAWHNEY AMARPREET S [US]) 30 January 2001 (2001-01-30) * figures 2A-2D * * column 1, line 65 - column 2, line 8 * * column 7, line 47 - line 52 * * column 9, line 12 - line 15 * * column 10, line 16 - line 33 * | 1-15 | INV. A61M11/00 A61M11/06 A61K38/00 A61M35/00 A61L27/00 |
| A | US 2013/253580 A1 (SUZUKI ZENETSU [JP] ET AL) 26 September 2013 (2013-09-26) * the whole document * | 1-15 | |
| A | CN 103 182 840 B (SHANGHAI FOCHIF MECHATRONICS TECHNOLOGY CO LTD) 28 January 2015 (2015-01-28) * the whole document * | 1-15 | |
| A | JORGENSEN ADAM M ET AL: "Decellularized Skin Extracellular Matrix (dsECM) Improves the Physical and Biological Properties of Fibrinogen Hydrogel for Skin Bioprinting Applications", NANOMATERIALS, vol. 10, no. 8, 29 July 2020 (2020-07-29), page 1484, XP093206038, ISSN: 2079-4991, DOI: 10.3390/nano10081484 * abstract * * Sections 2.1, 2.3 and 3 * | 11-15 | **TECHNICAL FIELDS SEARCHED (IPC)** A61K A61M A61L |
| A | US 2023/372575 A1 (MARCHAL CORRALES JUAN ANTONIO [ES] ET AL) 23 November 2023 (2023-11-23) * paragraphs [0112], [0258] - [0261] * | 11-15 | |

The present search report has been drawn up for all claims

| Place of search | Date of completion of the search | Examiner |
|---|---|---|
| Munich | 26 September 2024 | Schembri, Valentina |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

**ANNEX TO THE EUROPEAN SEARCH REPORT
ON EUROPEAN PATENT APPLICATION NO.**

EP 24 38 2365

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2024

| Patent document cited in search report | | Publication date | Patent family member(s) | | Publication date |
|---|---|---|---|---|---|
| US 6179862 | B1 | 30-01-2001 | ES | 2363922 T3 | 19-08-2011 |
| | | | US | 6179862 B1 | 30-01-2001 |
| | | | US | 6673093 B1 | 06-01-2004 |
| US 2013253580 | A1 | 26-09-2013 | CA | 2817098 A1 | 18-05-2012 |
| | | | CN | 103237509 A | 07-08-2013 |
| | | | CN | 107051764 A | 18-08-2017 |
| | | | EP | 2638868 A1 | 18-09-2013 |
| | | | JP | 5792452 B2 | 14-10-2015 |
| | | | JP | 2012100851 A | 31-05-2012 |
| | | | KR | 20130136482 A | 12-12-2013 |
| | | | US | 2013253580 A1 | 26-09-2013 |
| | | | WO | 2012063464 A1 | 18-05-2012 |
| CN 103182840 | B | 28-01-2015 | NONE | | |
| US 2023372575 | A1 | 23-11-2023 | EP | 4279093 A1 | 22-11-2023 |
| | | | ES | 2956802 A1 | 28-12-2023 |
| | | | US | 2023372575 A1 | 23-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017304600 A1

**Non-patent literature cited in the description**

- *TISSEEL*, 18 October 2022, https://globaladvanced-surgery.baxter.com/tisseel
- *ARTISS*, 18 October 2022, https://globaladvanced-surgery.baxter.com/artiss
- *VISTASEALTM Fibrin Glue & Sealant | Ethicon*, 18 October 2022, https://www.jnjmedtech.com/en-US/product/vistaseal-fibrin-sealant-human
- *Vivostat - Fibrin Sealant*, 18 October 2022, https://vi-vostat.com/products/vivostat-fibrin-sealant
- **O. CATANZANO** ; **M.C. STRACCIA** ; **A. MIRO** ; **F. UNGARO** ; **I. ROMANO** ; **G. MAZZARELLA** ; **G. SANTAGATA** ; **F. QUAGLIA** ; **P. LAURIENZO** ; **M. MALINCONICO**. Spray-by-spray in situ crosslinking alginate hydrogels delivering a tea tree oil microemulsion. *European Journal of Pharmaceutical Sciences*, 2015, vol. 66, 20-28, https://doi.org/10.1016/j.ejps.2014.09.018
- **P.M. NEUMANN** ; **B. ZUR** ; **Y. EHRENREICH**. Gelatin-based sprayable foam as a skin substitute. *J Biomed Mater Res.*, 1981, vol. 15, 9-18, https://doi.org/10.1002/jbm.820150105
- **Y. LI** ; **Q.Q. LENG** ; **X.L. PANG** ; **H. SHI** ; **Y.L. LIU** ; **S.S. XIAO** ; **L. ZHAO** ; **P. ZHOU** ; **S.Z. FU**. Therapeutic effects of EGF-modified curcumin/chitosan nano-spray on wound healing. *Regen Biomater.*, 2021, vol. 8, https://doi.org/10.1093/RB/RBAB009
- **J. CHEN** ; **H. WANG** ; **L. MEI** ; **B. WANG** ; **Y. HUANG** ; **G. QUAN** ; **C. LU** ; **T. PENG** ; **X. PAN** ; **C. WU**. A pirfenidone loaded spray dressing based on lyotropic liquid crystals for deep partial thickness burn treatment: healing promotion and scar prophylaxis. *J Mater Chem B.*, 2020, vol. 8, 2573-2588, https://doi.org/10.1039/C9TB02929J
- **K.M.G. JÁUREGUI** ; **J.C.C. CABRERA** ; **E.P.S. CENICEROS** ; **J.L.M. HERNÁNDEZ** ; **A. ILYINA**. A new formulated stable papin-pectin aerosol spray for skin wound healing. *Biotechnology and Bioprocess Engineering*, 2009, vol. 14, 450-456, https://doi.org/10.1007/s12257-008-0268-0
- **B. TER HORST** ; **R.J.A. MOAKES** ; **G. CHOUHAN** ; **R.L. WILLIAMS** ; **N.S. MOIEMEN** ; **L.M. GROVER**. A gellan-based fluid gel carrier to enhance topical spray delivery. *Acta Biomater.*, 2019, vol. 89, 166-179, https://doi.org/10.1016/j.actbio.2019.03.036

- **W.S. VEAZEY** ; **K.U. ANUSAVICE** ; **K. MOORE**. Mammalian cell delivery via aerosol deposition. *J Biomed Mater Res B Appl Biomater.*, 2005, vol. 72, 334-338, https://doi.org/10.1002/jbm.b.30159
- **F.A. NAVARRO** ; **M.L. STONER** ; **C.S. PARK** ; **J.C. HUERTAS** ; **H.B. LEE** ; **F.M. WOOD** ; **D.P. ORGILL**. Sprayed Keratinocyte Suspensions Accelerate Epidermal Coverage in a Porcine Microwound Model. *Journal of Burn Care & Rehabilitation*, 2000, vol. 21, 513-518, https://doi.org/10.1097/00004630-200021060-00007
- **F.O.G. FRAULIN** ; **A. BAHORIC** ; **A.R. HARROP** ; **T. HIRUKI** ; **H.M. CLARKE**. Autotransplantation of epithelial cells in the pig via an aerosol vehicle. *Journal of Burn Care and Rehabilitation*, 1998, vol. 19, 337-345, https://doi.org/10.1097/00004630-199807000-00012
- **J.C. GERLACH** ; **C. JOHNEN** ; **C. OTTOMAN** ; **K. BRÄUTIGAM** ; **J. PLETTIG** ; **C. BELFEKROUN** ; **S. MÜNCH** ; **B. HARTMANN**. Method for autologous single skin cell isolation for regenerative cell spray transplantation with non-cultured cells. *International Journal of Artificial Organs*, 2011, vol. 34, 271-279, https://doi.org/10.5301/IJAO.2011.6508
- **B. HARTMANN** ; **A. EKKERNKAMP** ; **C. JOHNEN** ; **J.C. GERLACH** ; **C. BELFEKROUN** ; **M. V. KÜNTSCHER**. Sprayed Cultured Epithelial Auto-grafts for Deep Dermal Burns of the Face and Neck. *Ann Plast Surg.*, 2007, vol. 58, 70-73, https://doi.org/10.1097/01.sap.0000250647.39784.bb
- **P. JOHNSTONE** ; **J.S.S. KWEI** ; **G. FILOBBOS** ; **D. LEWIS** ; **S. JEFFERY**. Successful application of keratinocyte suspension using autologous fibrin spray. *Burns*, 2017, vol. 43, e27-e30, https://doi.org/10.1016/j.burns.2016.05.010
- **R. ESTEBAN-VIVES** ; **M.S. CHOI** ; **M.T. YOUNG** ; **P. OVER** ; **J. ZIEMBICKI** ; **A. CORCOS** ; **J.C. GERLACH**. Second-degree burns with six etiologies treated with autologous noncultured cell-spray grafting. *Burns*, 2016, vol. 42, e99-e106, https://doi.org/10.1016/j.burns.2016.02.020
- *AVITA MEDICAL*, 13 October 2022, https://avitame-dical.com/product

- *Our Technology | RenovaCare*, 17 October 2022, https://www.renovacareinc.com/technology